# EUROPÄISCHE PATENTSCHRIFT

(11) **EP 4 181 851 B1**
(45) Veröffentlichungstag und Bekanntmachung des Hinweises auf die Patenterteilung: **24.09.2025**
(21) Anmeldenummer: 21749553.0
(22) Anmeldetag: 15.07.2021
(51) Int. Cl.: A61G 10/00, A61G 13/10

(54) **SYSTEM ZUM SCHUTZ VOR LUFTGETRAGENEN KRANKHEITSERREGERN IN EINEM OPERATIONSUMFELD**
SYSTEM FOR PROTECTION AGAINST AIRBORNE PATHOGENS IN A SURGICAL ENVIRONMENT
SYSTÈME DE PROTECTION CONTRE DES PATHOGÈNES AÉRIENS DANS UN ENVIRONNEMENT CHIRURGICAL

(30) Priorität: 16.07.2020 DE 102020118863
(43) Veröffentlichungstag der Anmeldung: 24.05.2023
(73) Patentinhaber: Reymond, Marc, 3145 Niederscherli (CH)
(72) Erfinder: Reymond, Marc, 3145 Niederscherli (CH)
(74) Vertreter: Witte, Weller & Partner Patentanwälte mbB
(86) Internationale Anmeldenummer: PCT/EP2021/069826
(87) Internationale Veröffentlichungsnummer: WO 2022/013382

(56) Entgegenhaltungen:
- WO-A1-2018/234803
- DE-T2- 69 423 367
- US-A- 3 820 536
- US-A1- 2017 273 845

## Beschreibung

Die vorliegende Erfindung betrifft ein System zum Schutz von medizinischem Personal und/oder eines Patienten vor luftgetragenen Krankheitserregern in einem Operationsumfeld und ein Verfahren zum Schutz von medizinischem Personal und/oder eines Patienten vor luftgetragenen Krankheitserregern in einem Operationsumfeld.

Während chirurgischer Eingriffe ist das medizinische Personal besonderen Infektionsrisiken ausgesetzt. Bei den Eingriffen kann es zur Ausscheidung von großen Mengen von Krankheitserregern kommen, sofern es sich bei dem Patienten um eine infizierte Person handelt.

Die Kontamination des medizinischen Personals durch Flüssigkeiten kann durch Schutzkleidung, Handschuhe und Gläser angemessen verhindert werden. Im Gegensatz dazu scheint das Infektionsrisiko durch virushaltige Aerosole sehr hoch zu sein. In früheren Arbeiten wurde das Vorhandensein verschiedener Krankheitserreger in chirurgischem Rauch nachgewiesen, einschließlich Corynebacterium, humanem Papillomavirus (HPV), Poliovirus, humanem Immunschwächevirus (HIV) und Hepatitis-Viren.

Luftgetragene Krankheitserreger stellen eine besondere Herausforderung dar. Hierbei handelt es sich um krankmachende Partikel oder Agenzien, wie bspw. Viren oder Bakterien, die eine längere Zeit in der Raumluft schweben, bspw. in einem Aerosol, und, anders als bei Flüssigkeitströpfchen, nach dem Ausscheiden, bspw. über die Atmung oder den Husten eines infizierten Patienten, nicht sofort zu Boden sinken.

Zu den luftgetragenen Krankheitserregern zählt das SARS-CoV-2 (Abk. englisch für 'Severe Acute Respiratory Syndrome Coronavirus 2'). Bis zum Mai 2020 waren laut dem Robert-Koch-Institut (RKI) in Deutschland 10'000 medizinische Fachkräfte mit SARS-CoV-2 infiziert; 16 von ihnen verstarben an der durch das SARS-CoV-2 verursachten Erkrankung COVID-19.

Die Quellen und Wege einer Kontamination mit SARS-CoV-2 sind nicht im Detail aufgeklärt. Es steht jedoch außer Zweifel, dass Kontakte mit Blut, Fäkalien, der Magen-Darm-Schleimhaut, Peritonealflüssigkeit wichtige Rollen spielen. Auch wird vermutet, dass SARS-CoV-2 im chirurgischen Rauch enthalten ist; Mowbray et al. (2020), Safe management of surgical smoke in the age of COVID-19, British Journal of Surgery, doi:101002/bjs. 11679.

Die derzeitigen in den Operationsräumen (ORs) eingesetzten Systeme zu Schutz des medizinischen Personals vor Infektion, insbesondere luftgetragenen Infektionen, bieten nur unzureichenden Schutz. Insbesondere ist unklar, wie effektiv aktuelle OR-Luftfiltersysteme (gemäß ISO 14644-1 Klasse ≤ 5) Partikel der Größe von SARS-CoV-2 mit einem Durchmesser von 60 bis 140 nm aus der Luft entfernen können.

Dementsprechend besteht ein dringender Bedarf an Systemen und Methoden zur Minderung der Risiken für das Gesundheitspersonal und des Patienten bei operativen Eingriffen aufgrund von luftgetragenen Krankheitserregern.

Der Erfindung liegt deshalb die Aufgabe zugrunde, ein System und Verfahren zum Schutz von medizinischem Personal und/oder eines Patienten vor luftgetragenen Krankheitserregern in einem Operationsumfeld bereitzustellen, das im Vergleich zu herkömmlichen Systemen die Gefahr von Infektion des Personals und/oder des Patienten verhindert oder zumindest reduziert.

Diese Aufgabe wird durch ein System gelöst, das folgendes aufweist:
- eine erste Elektrode, die zur Anbringung an das medizinische Personal ausgestaltet ist,
- eine zweite Elektrode, die zur Anbringung an den Patienten ausgestaltet ist,
- einen ersten Generator zur Beaufschlagung der Elektroden mit Spannung,
wobei die erste und/oder zweite Elektrode zur Ionisierung der luftgetragenen Krankheitserreger außerhalb der Körpers des Patienten eingerichtet ist, und wobei die erste und die zweite Elektrode bei Beaufschlagung mit Spannung die gleiche Polarität aufweisen.

Sie wird ferner durch ein Verfahren gelöst, das folgende Schritte aufweist:
1. Anbringen einer ersten Elektrode an das medizinische Personal,
2. Anbringen einer zweiten Elektrode an den Patienten,
3. Beaufschlagen der ersten und zweiten Elektrode mit Spannung derart, dass die luftgetragenen Krankheitserreger außerhalb des Körpers des Patienten ionisiert werden, wobei die erste und die zweite Elektrode die gleiche Polarität, vorzugsweise negative Polarität, aufweisen.

Das erfindungsgemäße Verfahren wird vorzugsweise unter Verwendung des erfindungsgemäßen Systems durchgeführt.

Die Erfinder haben erkannt, dass die im Stand der Technik grundsätzlich bekannte Technologie der sogenannten elektrostatischen Präzipitation zielgerichtet dazu eingesetzt werden kann, um während eines operativen Eingriffs das Risiko einer Infektion des medizinischen Personals und/oder des Patienten mit luftgetragenen Krankheitserregen zu minimieren, ggf. sogar zu vermeiden. Dazu wird unter Einsatz von Elektroden, bspw. Bürstenelektroden, die außerhalb des Körpers des Patienten und des medizinischen Personals angebracht werden, Spannungen gleicher Polarität erzeugt. Durch die erste oder zweite Elektrode werden die luftgetragenen Krankheitserreger außerhalb des Körpers des Patienten ionisiert und mit einer Ladung einer solchen Polarität versehen, die der Polarität der Ladung entspricht, die durch die Elektroden an dem medizinischen Personal und/oder dem Patienten erzeugt wird. Erfindungsgemäß weisen die erste und die zweite Elektrode bei Beaufschlagung mit Spannung durch den Generator die gleiche Polarität auf, also negative oder positive Polarität.

Durch die gleichgerichtete Ladung bzw. Polarität der ionisierten Krankheitserreger und des medizinischen Personals bzw. Patienten wird durch die elektrostatische Abstoßung eine Kontamination mit dem Krankheitserreger verhindert.

Die erste Elektrode wird an einem geeigneten Ort am medizinischen Personal angebracht, bspw. an der Brust (z.B. als oder im Kragen), dem Kopf (z.B. an einem Helm oder einer Kappe), Gesicht (z.B. integriert in eine Schutzmaske) etc. Die zweite Elektrode wird an geeigneter Stelle an dem Patienten angebracht, z.B. in unmittelbarer Nähe des Operationsgebietes und/oder an oder in der Nähe des Munds und/oder der Nase des Patienten.

In einer Ausführungsform der Erfindung kann es sich um ein mobiles System handeln.

Bei der elektrostatischen Präzipitation kommt es zur Freisetzung von Ladungsträgern in ein Gas wie der Raumluft, i.d.R. von Elektronen. Dies führt zur Aufladung von in dem Gas befindlichen Partikeln im elektrischen Feld.

Die in der Erfindung zum Einsatz kommende elektrostatische Präzipitation wird im Stand der Technik in sogenannten Elektrofiltern eigesetzt, die der Entfernung von Feinstaub aus Abgasen dienen, welche beispielsweise bei der kommerziellen Herstellung von Zement und Papier entstehen. Dort werden die geladenen Staubteilchen zu einer Niederschlagselektrode transportiert, an der sie anhaften. Von der Niederschlagselektrode lassen sich die Teilchen als Staubschicht entfernen.

In der Medizin wird die elektrostatische Präzipitation zur Ionisierung von Partikeln im chirurgischen Rauch eingesetzt; siehe Mowbray et al. (a.a.O.). Anders als bei dem erfindungsgemäßen System werden dabei negative Ionen innerhalb des Körpers, nämlich in der Bauchhöhle erzeugt. Diese Ionen verleihen bei dem bekannten System den Partikeln des chirurgischen Rauches eine vorübergehende negative Ladung. Die elektrostatisch geladenen Partikel werden aufgrund des Vorhandenseins der während der Operation verwendeten Standard-Patientenrückführungselektrode vom Patientengewebe angezogen. Die Ladung wird neutralisiert, wenn die Partikel auf der Oberfläche der Peritonealwand ausfallen. Eine bei dem bekannten System eingesetzte kommerziell erhältliche Vorrichtung ist unter dem Namen Ultravision^{™} bekannt, die in der WO 2018/234803 näher beschreiben ist. Das bekannte System hat jedoch den Nachteil, dass es für Operationen, bei denen kein chirurgischer Rauch entsteht, da bspw. keine Hitzeeinwirkung stattfindet, zum Schutz des Personals und/oder des Patienten nicht geeignet ist. Auch bietet das bekannte System keinen hinreichenden Schutz vor luftgetragenen Krankheitserreger, die sich außerhalb des Körpers des Patienten bzw. medizinischen Personals befinden.

Die Erfindung schafft hier Abhilfe. Sie bietet sowohl für das medizinische Personal Schutz vor luftgetragenen Krankheitserregern, die von dem Patienten oder Mitgliedern des Operationsteams ausgeschieden werden, als auch für den Patienten vor luftgetragenen Krankheitserregern, die von dem medizinischen Personal ausgeschieden werden.

Nach einer Ausführungsform der Erfindung sind die luftgetragenen Krankheitserreger Viren, vorzugsweise SARS-CoV-2.

Diese Maßnahme hat den Vorteil, dass eine Anpassung des erfindungsgemäßen Systems an besonders relevante pandemisch auftretende Krankheitserreger erfolgt. Besonders in Pandemien ist es für eine erfolgreiche Bekämpfung der Verbreitung des Erregers entscheidend, dass das behandelnde medizinische Personal vor Infektionen geschützt wird. Das erfindungsgemäße System stellt damit ein wirksames Werkzeug zur Eindämmung pandemisch auftretender Viruserkrankungen dar.

Nach einer weiteren Ausführungsform der Erfindung weisen die erste und die zweite Elektrode bei Beaufschlagung mit Spannung negative Polarität auf.

Diese Maßnahme hat den Vorteil, dass eine Anpassung an im Bereich der Medizin zum Einsatz kommende Elektroden und Systeme, wie Ultravision^{™} (Alesi Surgical Cardiff, Vereinigtes Königreich), erfolgt. Die Herstellung des erfindungsgemäßen Systems wird dadurch erleichtert und die Kosten verringert.

Nach einer Ausführungsform der Erfindung ist die erste Elektrode zur Anbringung an dem oder in der Nähe des Kopfes des medizinischen Personals ausgestaltet.

Die Maßnahme hat den Vorteil, dass im Bereich der oberen Atemwege, also dem Mund und der Nase, den Haupteinfallstoren luftgetragener Krankheitserreger, Ladungen erzeugt werden, die durch elektrostatische Abstoßung eine Annäherung der Erreger verhindern. Die Anbringung kann dabei am Kopf oder in der Nähe, z.B. dem Hals, erfolgen.

Es versteht sich, dass auch die zweite Elektrode im Bereich des Kopfes, entsprechend der ersten Elektrode angebracht werden kann. Dadurch bilden sich auch an dem Patienten, bspw. im Bereich des Mundes und/oder der Nase, Ladungsträger mit einer Polarität, die der der luftgetragenen Krankheitserreger entspricht. Die Aufnahme der Krankheitserreger durch den Patienten über die oberen Atemwege wird dadurch wirksam verhindert.

Nach einer noch weiteren Ausführungsform der Erfindung ist die erste Elektrode in ein Stirnband, eine Kappe, ein Visier und/oder ein Halsband integriert.

Diese Maßnahme hat den Vorteil, dass die konstruktiven Voraussetzungen für eine Anbringung der ersten Elektrode an dem Kopf des medizinischen Personals geschaffen werden.

Nach einer werteren Ausführungsform der Erfindung ist die zweite Elektrode zur Anbringung an oder in der Nähe einer Operationsstelle an dem Patienten ausgestaltet.

Diese Maßnahme hat den Vorteil, dass von dem Patienten ausgeschiedene luftgetragene Krankheitserreger unmittelbar am Ort ihrer Freisetzung ionisiert werden und eine unkontrollierte Verbreitung über die Luft verhindert wird. Erfindungsgemäß erfordert eine Anbringung an oder in der Nähe einer Operationsstelle eine Beabstandung, die klein genug ist, um die effektive Ionisierung von aus der Operationsstelle austretenden luftgetragenen Krankheitserregern zu gewährleisten.

In einer weiteren Ausführungsform der Erfindung ist die zweite Elektrode in ein Operationstuch integriert, vorzugsweise im Bereich der Operationsöffnung.

Diese Maßnahme hat den Vorteil, dass eine unmittelbare Ionisierung von aus der Operationsstelle (z.B. Abdomen) austretenden luftgetragenen Krankheitserregern sichergestellt wird. Besonders geeignet sind sogenannte Bürstenelektroden ("velcro" style), die sich in ein Operationstuch einnähen lassen, bspw. im Umfangsbereich der Operationsöffnung.

In einer weiteren Ausführungsvariante weist das erfindungsgemäße System eine Absaugvorrichtung zum Absaugen der luftgetragenen Krankheitserreger, insbesondere der ionisierten luftgetragenen Krankheitserreger auf.

Diese Maßnahme hat den Vorteil, dass das erfindungsgemäße System um eine Komponente erweitert wird, die die sichere Entfernung der luftgetragenen Krankheitserreger vom Operationsort gewährleistet. Ein geeignetes Absaugsystem umfasst die sogen. frontale Vortex-Absaugung. Die Absaugvorrichtung kann in geeigneter Art und Weise im System angeordnet werden, um eine maximale Clearance der Krankheitserreger zu erreichen, bspw. zwischen dem Patienten und dem medizinischen Personal. Die Strömung kann bspw. bei 300 - 500 m³/h pro Absaugvorrichtung liegen. Bei der frontalen Vortex-Absaugvorrichtung kann es sich Einmalgeräte handeln, die vorzugsweise steril sind. In einer Ausführungsform kann eine solche Vorrichtung typischerweise Abmessungen von etwa 60 x 10 cm aufweisen. Die Luftgeschwindigkeit kann, in einer Ausführungsform, ca. 16 m/s betragen. In einer weiteren Ausführungsform kann die Absaugvorrichtung unter Unterdruck gehalten werden, bis sie zur sicheren Entsorgung in hermetische Behälter, z. B. Plastiktüten mit Verschluss, gegeben werden. Die Absaugrichtung kann in einer Ausführungsform an einem Operationstisch befestigbar sein. Alternativ oder zusätzlich kann die Absaugvorrichtung in das Operationstuch integriert werden, um die Sicherheit der Handhabung zu erleichtern und zu erhöhen. In einer weiteren Ausführungsform handelt es sich bei der Absaugvorrichtung um ein mobiles Gerät. In einer weiteren Ausführungsform ist die Absaugvorrichtung mit einer Abdeckung, vorzugsweise einer zirkumferentiellen Abdeckung, versehen, die einen Luftaustritt in die Umgebung minimiert.

In einer Weiterbildung der Erfindung ist die Absaugvorrichtung mit Spannung beaufschlagbar, wobei vorzugsweise die Spannung eine Polarität aufweist, die der Polarität der ersten und/oder zweiten Elektrode entgegengesetzt ist.

Diese Maßnahme hat den Vorteil, dass mittels elektrostatischer Wechselwirkung eine zusätzliche Auslenkung der ionisierten luftgetragenen Krankheitserreger in Richtung der Absaugvorrichtung erfolgt. Die Beaufschlagung der Absaugvorrichtung kann durch den Generator erfolgen, der auch die Elektroden mit Spannung versorgt oder durch einen separaten zweiten Generator. Die Beaufschlagung der Absaugvorrichtung kann erfindungsgemäß in einer Ausgestaltung über eine dritte Elektrode erfolgen, die zur Anbringung an die Absaugvorrichtung ausgestaltet ist. Die dritte Elektrode ist mit dem ersten oder einem weiteren Generator verbindbar.

Das erfindungsgemäße Verfahren weist deshalb in einer Ausführungsform folgenden weiteren Schritt auf:
4. Absaugen der ionisierten luftgetragenen Krankheitserreger, vorzugsweise über eine Absaugvorrichtung, die bei Beaufschlagung mit Spannung eine Polarität aufweist, die der Polarität der ersten und/oder zweiten Elektrode entgegengesetzt ist.

In einer Ausführungsform der Erfindung weist das System zusätzlich eine Vorrichtung zur Emittierung von UV-C-Strahlung auf.

Diese Maßnahme integriert eine zusätzlich Komponente, die für erhöhte Sicherheit sorgt. Kurzwelliges Licht mit einer Wellenlänge von 250 nm bis 270 nm liefert Aktivierungsenergie zur Anregung einer photochemischen Reaktion, die auf Krankheitserreger, insbesondere Viren, einen zytotoxischen Effekt hat. UV-C-Beleuchtung ist eine schnelle, kostengünstige, vergleichsweise wenig toxische Methode. Die Vorrichtung zur Emittierung von UV-C-Strahlung ist bspw. in das Absaugsystem integriert. In einer weiteren Ausführungsform wird eine bestimmte Anzahl von UV-C-Lichtquellen, typischerweise 4 Quellen, optional mit einer Leistung von jeweils 160 W, optional mit einer Wellenlänge von 254 nm, optional ozonfrei, bevorzugt in Längsposition in einem, zwei oder mehr zylindrischen Reaktoren angeordnet, um die Expositionszeit auf das UV-C-Licht und die photodynamische Toxizität zu erhöhen.

In einer noch weiteren Ausführungsform der Erfindung weist das System ferner eine Filtervorrichtung, vorzugsweise einen Schwebstofffilter (HEPA) und/oder Hochleistungs-Schwebstofffilter (ULPA) auf.

Diese weitere Komponente sorgt für eine zusätzliche Dekontaminationswirkung. Ultralow Particle Air (ULPA) Filter halten 99,9% von Partikeln mit 100 nm Durchmesser zurück, so dass sie die meisten SARS-CoV-2-Viruspartikel einfangen können. Die Filtervorrichtung ist in geeigneter Art und Weise im System angeordnet, bspw. in der Decke des Operationsraums. Der Luftfluss kann nach Bedarf adjustiert werden und liegt üblicherweise im Bereich von 10m³/min und 50 m³/min.

In einer Ausführungsform der Erfindung weist das System eine Vorrichtung zur Zufuhr von Desinfektionsmittel, vorzugsweise H₂O₂ und/oder Formaldehyd, auf.

Auch diese zusätzliche Komponente erhöht die Sicherheit und den Schutz des Personals und Patienten vor einer Infektion mit luftgetragenen Krankheitserregern. Insbesondere wird das Risiko der Handhabung einer kontaminierten Filtervorrichtung minimiert. Die Vorrichtung zur Zufuhr von Desinfektionsmittel ist in geeigneter Art und Weise im System angeordnet.

Das System kann ferner weitere Komponenten aufweisen, wie bspw. einen Aktivkohlefilter, der optional in einer Zugangsöffnung des Systems platzierbar und aus dieser entnehmbar ist.

Die Merkmale, Ausführungsformen und Vorteile des erfindungsgemäßen Systems gelten für das erfindungsgemäße Verfahren entsprechend.

Weitere Vorteile und Merkmale ergeben sich aus der nachfolgenden Beschreibung bevorzugter Ausführungsformen und den beigefügten Zeichnungen.

Es versteht sich, dass die vorstehend genannten und nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

In den beiliegenden Zeichnungen zeigen:
- Fig. 1: eine Ausführungsform des erfindungsgemäßen Systems;
- Fig. 2: einen Ausschnitt der in der Fig. 1 dargestellten Ausführungsform;
- Fig. 3: eine Ausführungsform der zweiten Elektrode, die in ein Operationstuch integriert ist;
- Fig. 4: Ausführungsformen der ersten Elektrode zur Befestigung an dem Körper des medizinischen Personals.

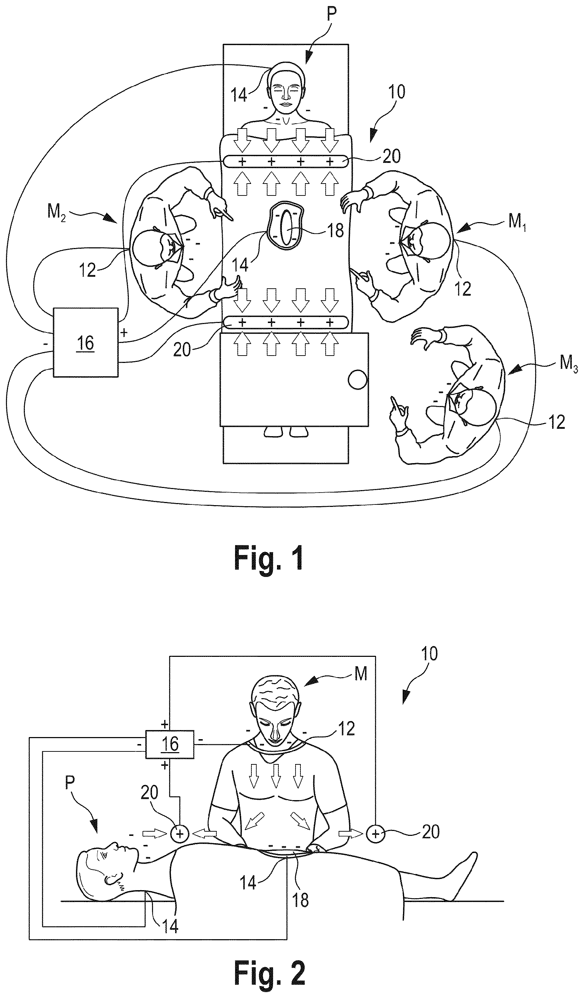

In der Fig. 1 ist das erfindungsgemäße System zum Schutz von medizinischem Personal und/oder eines Patienten mit dem Bezugszeichen 10 versehen. Das medizinische Personal wird mit M₁, M₂, M₃ und der Patient mit P dargestellt. Das System 10 umfasst eine erste Elektrode 12, die zur Anbringung an das medizinische Personal M₁, M₂, M₃ ausgestaltet ist, eine zweite Elektrode 14, die zur Anbringung an den Patienten P ausgestaltet ist, und einen Generator 16 zur Beaufschlagung der Elektroden 12, 14 mit Spannung. In der dargestellten Ausführungsform ist die zweite Elektrode 14 sowohl im Kopfbereich des Patienten P als auch an der Operationsstelle 18 angebracht. Bei der Beaufschlagung der Elektroden 12, 14 mit Spannung über den Generator 16 weisen beide Elektroden 12, 14 die gleiche, in der dargestellten Ausführungsform negative Polarität auf. Beide Elektroden 12, 14 erzeugen dadurch negativ geladene Ionen in unmittelbarer Umgebung, bspw. in Gesichtsnähe des medizinischen Personals M₁, M₂, M₃, und an dem Patienten P, dort bspw. ebenfalls in Gesichtsnähe und/oder der Operationsstelle 18. An der Operationsstelle 18 verleihen die negativ geladenen Ionen, erzeugt durch die zweite Elektrode 14, den luftgetragenen Krankheitserregern, bspw. SARS-CoV-2, die während des operativen Eingriffes freigesetzt werden können, eine negative Ladung. Negative Ladungen sind durch Minuszeichen (- - -) dargestellt. Aufgrund der negativen Ladungen der luftgetragenen Krankheitserreger und des medizinischen Personals M₁, M₂, M₃ werden die luftgetragenen Krankheitserreger von letzterem ferngehalten. Dadurch wird eine Infektion des medizinischen Personals verhindert.

In der dargestellten Ausführungsform ist eine Absaugvorrichtung 20 dargestellt. Diese ist mit dem Generator 16 verbunden, der an der Absaugvorrichtung eine Spannung positiver Polarität erzeugt, dargestellt durch Pluszeichen (+++). Die Absaufvorrichtung 20 saugt Umgebungsluft und chirurgischen Rauch ein, einschließlich freigesetzte luftgetragene Krankheitserreger, was aufgrund der entgegengesetzten Ladungen durch elektrostatische Wechselwirkung begünstigt wird. Der Luftstrom ist durch die dicken Pfeile dargestellt. Die Krankheitserreger werden dadurch aus dem Operationsumfeld entfernt.

Es versteht sich, dass mit dem erfindungsgemäßen System auch eine Infektion des Patienten P verhindert wird, sollte das medizinische Personal M₁, M₂, M₃ Viren ausscheiden. Diese würden durch die erste Elektrode 12 negativ ionisiert und aufgrund der negativen Ladung am Patienten P durch elektrostatische Abstoßung von letzterem ferngehalten.

Es versteht sich ferner, dass die Ladungsverhältnisse, so wie sie in der Fig. 1 dargestellt sind, umgekehrt ausgebildet sein können, also positive Ladungen an dem medizinischen Personal M₁, M₂, M₃, dem Patienten P und der Operationsstelle 18 bzw. den luftgetragenen Krankheitserregern, und negative Ladungen an der Absaugvorrichtung 20.

In der Fig. 2 ist ein Ausschnitt des erfindungsgemäßen Systems 10 zum Schutz von medizinischem Personal M und/oder eines Patienten P dargestellt. Die erste Elektrode 12 ist im Kopfbereich des Patienten P und an der Operationsstelle 18 angebracht und mit dem Generator 16 verbunden. Die zweite Elektrode 14, die ebenfalls von dem Generator 16 gespeist wird, ist mit dem medizinischen Personal M verbunden und in Form eines Halsbandes ausgestaltet. Die in der Nähe der Operationsstelle 18 ggf. auftretenden Viren sowie der chirurgische Rauch, der Bereich um das Gesicht des medizinischen Personals P und der Bereich um das Gesicht des Patienten P weisen negative Ladungsträger auf (- - -). Die mit dem Generator 16 verbundene Absaugvorrichtung 20 ist positiv geladen (+++). Die negativ geladenen luftgetragenen Krankheitserreger werden aufgrund der positiven Ladung der Absaugvorrichtung 20 in letztere eingesogen. Der Luftstrom ist durch die dicken Pfeile dargestellt.

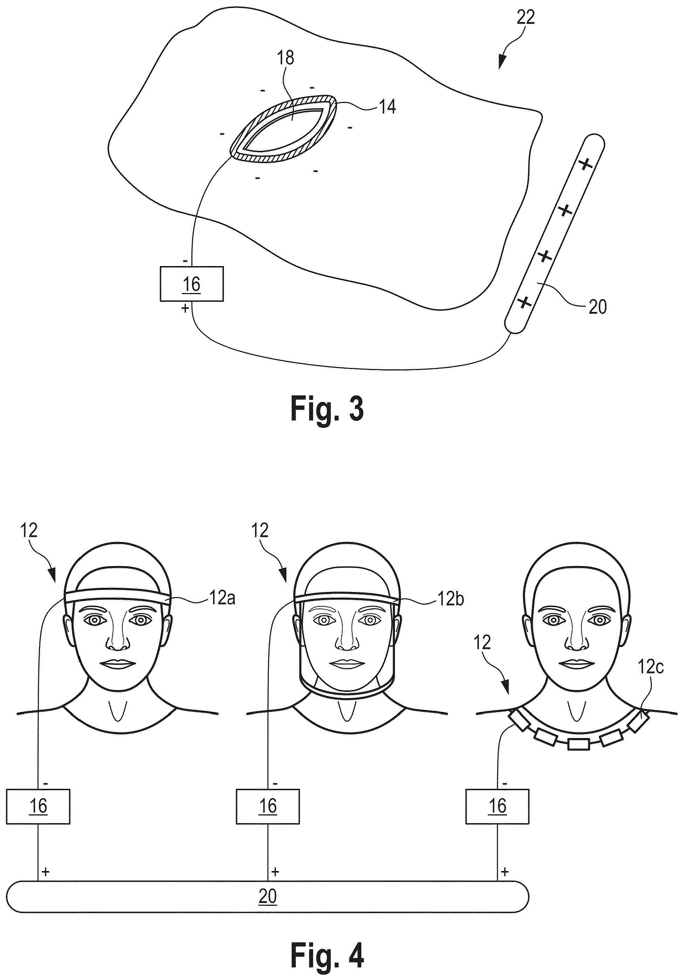

In der Fig. 3 ist eine Ausführungsvariante der zweiten Elektrode 14 dargestellt, bei der letztere in ein Operationstuch 22 integriert ist, und zwar in einer Öffnung in dem Operationstuch 22, die bei Gebrauch über der Operationsstelle 18 angeordnet wird. Die zweite Elektrode wird über den Generator 16 mit Spannung beaufschlagt und weist in der dargestellten Variante negative Polarität auf. Dargestellt ist ferner die Absaugvorrichtung 20, die über den Generator 16 mit Spannung beaufschlagt wird und in der gezeigten Variante positive Polarität aufweist.

In der Fig. 4 sind verschiedenen Ausführungsformen der ersten Elektrode 12 dargestellt, nämlich in Form eines Stirnbandes 12a, integriert in ein Gesichtsvisier 12b und in Form einer Halskette 12c. Dargestellt sind ferner der Generator 16 sowie die Absaugvorrichtung 20. Der Generator beaufschlagt die erste Elektrode 12, 12a, 12b, 12c mit Spannung, so dass letztere negativ geladen ist (- - -), er beaufschlagt die Absaugvorrichtung mit Spannung, so dass diese positiv geladen ist (+++).

## Patentansprüche

1. System (10) zum Schutz von medizinischem Personal (M) und/oder eines Patienten (P) vor luftgetragenen Krankheitserregern in einem Operationsumfeld, das folgendes aufweist:
- eine erste Elektrode (12), die zur Anbringung an das medizinische Personal (M) ausgestaltet ist,
- eine zweite Elektrode (14), die zur Anbringung an den Patienten (P) ausgestaltet ist,
- einen ersten Generator (16) zur Beaufschlagung der Elektroden (12, 14) mit Spannung,
wobei die erste und/oder zweite Elektrode (12, 14) zur Ionisierung der luftgetragenen Krankheitserreger außerhalb der Körpers des Patienten (P) eingerichtet ist, und wobei die erste und die zweite Elektrode (12, 14) bei Beaufschlagung mit Spannung die gleiche Polarität aufweisen.

2. System nach Anspruch 1, **dadurch gekennzeichnet, dass** die luftgetragenen Krankheitserreger Viren, vorzugsweise SARS-CoV-2 sind.

3. System nach einem der Ansprüche 1 oder 2, **dadurch gekennzeichnet, dass** die erste und die zweite Elektrode (12, 14) bei Beaufschlagung mit Spannung negative Polarität aufweisen.

4. System nach einem der vorherigen Ansprüche, **dadurch gekennzeichnet, dass** die erste Elektrode (12) zur Anbringung an dem oder in der Nähe des Kopfes des medizinischen Personals (M) ausgestaltet ist.

5. System nach einem der vorherigen Ansprüche, **dadurch gekennzeichnet, dass** die erste Elektrode (12) in ein Stirnband, eine Kappe, ein Visier und/oder ein Halsband integriert ist.

6. System nach einem der vorherigen Ansprüche, **dadurch gekennzeichnet, dass** die zweite Elektrode (14) zur Anbringung an oder in der Nähe einer Operationsstelle (18) an dem Patienten (P) ausgestaltet ist, wobei vorzugsweise die zweite Elektrode (14) in ein Operationstuch integriert ist, weiter vorzugsweise im Bereich der Operationsöffnung (18).

7. System nach einem der vorherigen Ansprüche, das ferner eine Absaugvorrichtung (20) zum Absaugen der ionisierten luftgetragenen Krankheitserreger aufweist.

8. System nach Anspruch 7, **dadurch gekennzeichnet, dass** die Absaugvorrichtung (20) mit Spannung beaufschlagbar ist.

9. System nach Anspruch 8, **dadurch gekennzeichnet, dass** die Absaugvorrichtung (20) bei Beaufschlagung mit Spannung eine Polarität aufweist, die der Polarität der ersten und/oder zweiten Elektrode (12, 14) entgegengesetzt ist.

10. System nach einem der vorherigen Ansprüche, das ferner eine Vorrichtung zur Emittierung von UV-C-Strahlung aufweist.

11. System nach einem der vorherigen Ansprüche, das ferner eine Filtervorrichtung, vorzugsweise einen Schwebstofffilter (HEPA) und/oder Hochleistungs-Schwebstofffilter (ULPA) aufweist.

12. System nach einem der vorherigen Ansprüche, das ferner eine Vorrichtung zur Zufuhr von Desinfektionsmittel, vorzugsweise H₂O₂ und/oder Formaldehyd, aufweist.

13. Verfahren zum Schutz von medizinischem Personal (M) und/oder eines Patienten (P) vor luftgetragenen Krankheitserregern, vorzugsweise SARS-CoV-2, in einem Operationsumfeld, das folgende Schritte aufweist:
1. Anbringen einer ersten Elektrode (12) an das medizinische Personal (M),
2. Anbringen einer zweiten Elektrode (14) an den Patienten (P),
3. Beaufschlagen der ersten und zweiten Elektrode (12, 14) mit Spannung derart, dass die luftgetragenen Krankheitserreger außerhalb des Körpers des Patienten (P) ionisiert werden, wobei die erste und die zweite Elektrode (12, 14) die gleiche Polarität, vorzugsweise negative Polarität, aufweisen.

14. Verfahren nach Anspruch 13, **dadurch gekennzeichnet, dass** es folgenden weiteren Schritt aufweist:
4. Absaugen der ionisierten luftgetragenen Krankheitserreger, vorzugsweise über eine Absaugvorrichtung (20), die bei Beaufschlagung mit Spannung eine Polarität aufweist, die der Polarität der ersten und/oder zweiten Elektrode (12, 14) entgegengesetzt ist.

15. Verfahren nach Anspruch 13 oder 14, **dadurch gekennzeichnet, dass** es unter Verwendung des Systems (10) nach einem der Ansprüche 1 bis 12 durchgeführt wird.

## Claims

1. A system (10) for protecting medical personnel (M) and/or a patient (P) from airborne pathogens in a surgical environment, comprising:
- a first electrode (12) configured for attachment to the medical personnel (M),
- a second electrode (14) configured for attachment to the patient (P),
- a first generator (16) for applying voltage to the electrodes (12, 14),
wherein the first and/or second electrode (12, 14) is adapted to ionize the airborne pathogens outside the patient's (P) body, and wherein the first and the second electrode (12, 14) exhibit the same polarity when voltage is applied.

2. The system according to claim 1, **characterized in that** the airborne pathogens are viruses, preferably SARS-CoV-2.

3. The system according to claim 1 or 2, **characterized in that** the first and the second electrode (12, 14) exhibit negative polarity when voltage is applied.

4. The system according to one of the preceding claims, **characterized in that** the first electrode (12) is configured for attachment on or near the head of the medical personnel (M).

5. The system according to one of the preceding claims, **characterized in that** the first electrode (12) is integrated into a headband, a cap, a visor and/or a neckband.

6. The system according to one of the preceding claims, **characterized in that** the second electrode (14) is configured for attachment to or near a surgical site (18) on the patient (P), wherein preferably the second electrode (14) is integrated into a surgical drape, more preferably in the region of the surgical opening (18).

7. The system according to one of the preceding claims, further comprising a suction device (20) for removing the ionized airborne pathogens.

8. The system according to claim 7, **characterized in that** the suction device (20) is capable of being supplied with voltage.

9. The system according to claim 8, **characterized in that** the suction device (20), when supplied with voltage, has a polarity opposite to the polarity of the first and/or second electrode (12, 14).

10. The system according to one of the preceding claims, further comprising a device for emitting UV-C radiation.

11. The system according to one of the preceding claims, further comprising a filter device, preferably a high-efficiency particulate air (HEPA) filter and/or an ultra-low penetration air (ULPA) filter.

12. The system according to one of the preceding claims, further comprising a device for supplying a disinfectant, preferably H₂O₂ and/or formaldehyde.

13. A method for protecting medical personnel (M) and/or a patient (P) from airborne pathogens, preferably SARS-CoV-2, in a surgical environment, comprising the steps of:
1. attaching a first electrode (12) to the medical personnel (M),
2. attaching a second electrode (14) to the patient (P),
3. applying voltage to the first and second electrode (12, 14) such that the airborne pathogens outside the patient's (P) body are ionized, wherein the first and second electrode (12, 14) exhibit the same polarity, preferably negative polarity.

14. The method according to claim 13, **characterized in that** it comprises the following further step:
4. removing the ionized airborne pathogens, preferably via a suction device (20), which, when supplied with voltage, exhibits a polarity opposite to the polarity of the first and/or second electrode (12, 14).

15. The method according to claim 13 or 14, **characterized in that** it is carried out using the system (10) according to one of claims 1 to 12.

## Revendications

1. Système (10) pour la protection d'un personnel médical (M) et/ou d'un patient (P) contre des agents pathogènes en suspension dans l'air dans un environnement chirurgical, présentant ce qui suit :
- une première électrode (12) conçue pour être appliquée sur le personnel médical (M),
- une seconde électrode (14) conçue pour être appliquée sur le patient (P),
- un premier générateur (16) permettant d'alimenter les électrodes (12, 14) en tension,
dans lequel la première et/ou la seconde électrode (12, 14) sont configurées pour ioniser les agents pathogènes en suspension dans l'air à l'extérieur du corps du patient (P), et dans lequel la première et la seconde électrode (12, 14) présentent la même polarité lorsqu'elles sont alimentées en tension.

2. Système selon la revendication 1, **caractérisé en ce que** les agents pathogènes en suspension dans l'air sont des virus, de préférence le SARS-CoV-2.

3. Système selon l'une des revendications 1 ou 2, **caractérisé en ce que** la première et la seconde électrode (12, 14) présentent une polarité négative lorsqu'elles sont alimentées en tension.

4. Système selon l'une des revendications précédentes,
**caractérisé en ce que** la première électrode (12) est conçue pour être appliquée sur la tête du personnel médical (M) ou à proximité de celle-ci.

5. Système selon l'une des revendications précédentes,
**caractérisé en ce que** la première électrode (12) est intégrée dans un serre-tête, une casquette, une visière et/ou un collier.

6. Système selon l'une des revendications précédentes,
**caractérisé en ce que** la seconde électrode (14) est conçue pour être appliquée sur le patient (P) au niveau d'un site opératoire (18) ou à proximité de celui-ci, dans lequel la seconde électrode (14) est de préférence intégrée dans un champ opératoire, plus préférentiellement dans la zone de l'ouverture opératoire (18).

7. Système selon l'une des revendications précédentes, présentant en outre un dispositif d'aspiration (20) permettant d'aspirer les agents pathogènes en suspension dans l'air et ionisés.

8. Système selon la revendication 7, **caractérisé en ce que** le dispositif d'aspiration (20) peut être alimenté en tension.

9. Système selon la revendication 8, **caractérisé en ce que** le dispositif d'aspiration (20) présente une polarité opposée à la polarité de la première et/ou de la seconde électrode (12, 14) lors d'une alimentation en tension.

10. Système selon l'une des revendications précédentes, présentant en outre un dispositif permettant l'émission d'un rayonnement UV-C.

11. Système selon l'une des revendications précédentes, présentant en outre un dispositif de filtration, de préférence un filtre pour matières en suspension dans l'air (HEPA) et/ou un filtre pour matières en suspension dans l'air à haute efficacité (ULPA).

12. Système selon l'une des revendications précédentes, présentant en outre un dispositif permettant l'apport en désinfectant, de préférence en H₂O₂ et/ou en formaldéhyde.

13. Procédé pour la protection d'un personnel médical (M) et/ou d'un patient (P) contre des agents pathogènes en suspension dans l'air, de préférence le SARS-CoV-2, dans un environnement chirurgical, présentant les étapes suivantes :
1. application d'une première électrode (12) sur le personnel médical (M),
2. application d'une seconde électrode (14) sur le patient (P),
3. alimentation en tension de la première et de la seconde électrode (12, 14) de telle sorte que les agents pathogènes en suspension dans l'air sont ionisés à l'extérieur du corps du patient (P), dans lequel la première et la seconde électrode (12, 14) présentent la même polarité, de préférence une polarité négative.

14. Procédé selon la revendication 13, **caractérisé en ce qu'**il présente l'étape supplémentaire suivante :
4. aspiration des agents pathogènes ionisés en suspension dans l'air, de préférence par l'intermédiaire d'un dispositif d'aspiration (20) qui, lors d'une alimentation en tension, présente une polarité opposée à la polarité de la première et/ou de la seconde électrode (12, 14).

15. Procédé selon la revendication 13 ou 14, **caractérisé en ce qu'**il est mis en œuvre à l'aide du système (10) selon l'une des revendications 1 à 12.
